# EUROPEAN PATENT SPECIFICATION

(11) **EP 1 868 585 B1**
(45) Date of publication and mention of the grant of the patent: **21.08.2019**
(21) Application number: 06726670.0
(22) Date of filing: 06.04.2006
(51) Int. Cl.: A61K 9/48

(54) **ABUSE RESISTANT CAPSULES**
GEGEN MISSBRAUCH GESICHERTE KAPSELN
CAPSULES A L'EPREUVE D'ABUS

(30) Priority: 06.04.2005 GB 0506982
(43) Date of publication of application: 26.12.2007
(73) Proprietor: MW Encap Limited, Livingston West Lothian EH53 0TH (GB)
(72) Inventor: YOUNG, Victor, Morrison, MW Encap Limited, Livingston, West Lothian EH53 0TH (GB)
(74) Representative: De Clercq & Partners
(86) International application number: PCT/GB2006/001267
(87) International publication number: WO 2006/106344

(56) References cited:
- EP-A2- 0 152 292
- WO-A-95/20947
- WO-A-97/48386
- WO-A-02/092060
- WO-A-2004/105735
- WO-A2-02/00206
- US-A1- 2004 058 946
- US-A1- 2005 191 244
- US-B1- 6 365 181

## Description

The present invention relates to the field of abuse resistant capsules. In addition, the present invention relates to a method for preparing abuse resistant capsules, see e.g. US2004/0058946 A1, which pertains to abuse deterrent formulations.

It should be noted that in the context of the present specification the term "modifier" should be understood to mean any compound or material that alters the properties of the abuse resistant capsules to which said modifier is added.

The abuse of pharmaceutical medicaments has now become a high profile topic. This abuse often takes the form of extracting, injecting, chewing or snorting different prescription drugs.

The continued abuse of pharmaceutical medicaments has led to regulatory authorities taking action. In particular, the abuse of temazepam soft gels has resulted in its 'delisting' by the Department of Health, thereby eliminating most of the UK market for temazepam manufacturers. The FDA currently appears to be considering firm action over the abuse of opiate medication in the US, and it is likely that this type of response will continue unless manufacturers take meaningful action.

A further problem associated with pharmaceutical medicaments is that they can be employed in spiking of drinks in other words so-called "drug rape" and "drug robbery". In particular, the misuse of drugs such as flunitrazepam has become a major problem. Incidents where fast dissolving tablets or powders containing hypnotic type compounds are secretly added to drinks in pubs and clubs are attracting major (and increasing) media attention. Current attempts to overcome such abuse by dyeing tablets are generally useless as the opportunity to spike drinks is typically taken in dimly lit clubs and the colour of the liquid can be masked by dark mixers such as cola.

Different drug substances have different properties (narcotic, sedative etc) and it is unlikely that a single drug substance will be abused by all possible routes (e.g. the abuse of flunitrazepam is likely to be restricted to the covert abuse of others). Hence abuse resistance built into a dosage unit must be tailored to combat the potential routes of abuse for that drug substance.

It is imperative that manufacturers consider and seriously address the issue of misuse of dosage units.

Some of the most widely used current pharmaceutical medicaments are tablets, powder filled capsules and filled soft gel capsules.

Tablets are a popular way of administering drugs but are open to abuse as they are crushable and the active ingredient can therefore be easily absorbed, extracted, dissolved, ingested or snorted.

Some dosage units are formulated so that they maintain a slow sustained release. Such medicaments contain extremely high doses of active pharmaceutical agent that are released slowly over a long period of time. However, in tablet form this type of dosage unit can be abused by chewing and mixing with saliva, or by crushing, which destroys the sustained release effect and releases a large dose of the active pharmaceutical agent. Tablets offer a vehicle for high melting point pharmaceutical medicaments.

Another popular way of administering pharmaceutical medicaments, which is similar to using tablets, is to use capsules filled with powder. Powder filled capsules can incorporate small amounts of high melting point materials or waxes, but not to any great extent. This is principally due to the method that these capsules are manufactured. In particular, the powder needs to be able to flow such that it can be dispensed by machines when manufacturing the powder filled capsules. Therefore, the powder cannot be made too waxy, gel-like or sticky as it will not flow through the machinery and will not be able to be dispensed properly.

A further method employed for administering pharmaceutical medicaments is to use soft gel capsules that can be manufactured by injecting materials between sheets of wet gelatin. At present, soft gel capsule manufacturing is limited to processing at temperatures of 40°C and therefore are unsuitable for encasing liquefied high melting point materials. Moreover, the contents of soft gel capsules reliquefy when heated to 40°C (which is very close to body temperature) and therefore are easy to extract and inject, making them open to abuse.

All types of dosage unit are potentially subject to abuse. However, self-abusers tend to abuse sustained release formulations as these are designed to release drug substances over a prolonged period and thus have a much higher drug loading than dosage units designed for immediate release. Sustained (or prolonged) release formats (normally tablets) do have some inbuilt abuse resistance (as they are specifically designed to release drug substance slowly) but this is overcome immediately by powdering the dosage unit when the drug content can be extracted or absorbed extremely quickly due to the large surface area of the powdered material.

Therefore it would be desirable to mitigate at least some of the problems associated with the prior art.

Accordingly, it is an object of the present invention to provide abuse resistant capsules.

Disclosed is an abuse resistant capsule comprising a hard shell liquid filled with a pharmaceutical medicament and at least one modifier selected to prevent abuse of the pharmaceutical medicament.

The modifier has a high melting point greater than 50 °C. This prevents abuse of the pharmaceutical medicament as it will only melt at high temperature and will re-solidify when it comes into contact with a syringe at room temperature. The temperature at which it melts is too high to inject.

The modifier may be insoluble in liquid. This prevents abuse of the pharmaceutical medicament as it will not dissolve in drinks but will instead float.

The modifier is preferably insoluble in aqueous solvents.

The modifier is preferably insoluble in ethanol.

The modifier preferably has a density less than 1.

The modifier is a waxy substance. Advantageously this prevent abuse of the pharmaceutical medicament as it will neither melt at injectable temperatures, dissolve readily in water, or be crushable to a powder.

Optionally the hard shell outer capsule comprises gelatin.

Alternatively, the hard shell outer capsule comprises hydroxypropyl methylcellulose (HPMC), pullalan or other hard shell materials.

The modifier constitutes an excipient in the abuse resistant material.

In addition to the waxy substance specified in claim 1, the abuse resistant capsule may be filled with a plurality of modifiers.

Preferably such modifier is one or more of the materials selected from the following groups; consisting of:
a) thermosoftening pharmaceutical bases including waxes, poloxamers, macrogol glycerides, PEGs, glycerol monooleates or monostearates, hydrogenated or partially hydrogenated glycerides and hard fats such as beeswax, poloxamer 188, Gelucires™, polyethylene 6000, glycerol monostearate, hydrogenated palm kernel oil, hydrogenated cottonseed oil, Softisan™ 138, Gelucire 40/01™ hexadecan-1-ol
b) Thixotropes such as fumed silica and pulverised attapulgite
c) viscosity modifiers such as hydroxyl propyl methyl cellulose or Gellan gum™ to increase viscosity or the standard pharmaceutical or food grade oils such as fractionated coconut oil, soyabean oil etc to decrease viscosity.

The modifier may comprise a thixotrope.

Preferably the thixotrope is hydroxypropyl methylcellulose (HPMC).

Alternatively the thixotrope is fumed silica.

The modifier may comprise a digestible material.

The modifier may comprise a paste.

The modifier may have an unpleasant taste.

The modifier may be a dye.

The modifier may be a viscous material.

The modifier may comprise suspended solids.

The pharmaceutical medicament may be mixed directly with the modifier. The modifier and pharmaceutical medicament may also be incorporated in the abuse resistant capsule as particles suspended in additional modifiers.

Preferably the particles are 100 -2000 micron in size and are comprised of active ingredient formulated with a modifier or modifier. The particles may be uncoated or may be coated with materials known to the art of particle coating.

According to a second aspect of the present invention there is provided a method for preparing an abuse resistant capsule of the first aspect comprising the steps of:
preparing a pharmaceutical medicament;
adding at least one modifier; and
filling a hard shell with the pharmaceutical medicament and the at least one modifier;
wherein the modifier is selected to prevent abuse of the pharmaceutical medicament.

Preferably the method comprises the further step of heating the pharmaceutical medicament to above 50°C.

Preferably the hard shell is filled at a temperature of above 50°C.

Preferably the hard shell is liquid filled at a temperature of above 50°C.

Preferably the pharmaceutical medicament is a liquid at the filling temperature.

Preferably the at least one modifier is a liquid at the filling temperature.

Preferably the pharmaceutical medicament and the at least one modifier are liquids at the filling temperature.

The abuse resistant capsule technology involves the application of modifiers, excipients and their properties in a formulation designed to combat various areas of pharmaceutical abuse. The liquid filling of hard shells, or outer capsules (hard shell liquid fill), has been found to be very useful in this respect.

Hard shell liquid fill has inherent advantages when choosing a foundation from which to build in abuse resistance to a dosage unit. The excipients that may be used to confer abuse resistance may be used in other dosage forms (e.g. waxes in sustained release tablets) however, in hard shell liquid fill, they confer abuse resistance because they can be used in concentrations greatly exceeding that possible in other dosage unit forms (greater than 10%).

Excipients or modifiers can be used in combinations which both allow release of the active ingredient but also impart anti-abuse characteristics. These types of dosage forms have not been previously developed or produced.

Therefore, using the technology of the present invention it is possible to use modifiers which dissolve to form a viscous solution at low temperatures and are insoluble at high temperatures thus protecting against extraction and filtration at low temperatures (when the wax is solid and any solution obtained is viscous) and against high temperature extraction (when the wax is liquid but insoluble and the modifier is now insoluble).

Although hard shell liquid fill provides a good technology on which to build abuse resistant formulations, this is not obvious to users of hard shell liquid fill, as excipients need to be used in combinations which both allow release of the active ingredient, but also impart anti-abuse characteristics. These types of dosage forms are not known to have been developed nor produced by other hard shell liquid encapsulators.

For example, a drug substance can be encapsulated in a totally water insoluble wax excipient, which will provide excellent abuse resistance. Unfortunately, this will prevent the drug being released when ingested by a patient, and thus this formulation will not provide a viable dosage unit. Modifiers need to be added which will provide a route (e.g., "channels") through the wax which will permit release of the drug substance, however it contributes to the abuse resistant of the dosage unit if these modifiers also provide resistance to abusers. For example, it is possible to use modifiers which dissolve to form a viscous solution at low temperatures and are insoluble at high temperatures, thus protecting against extraction and filtration at low temperatures (when the wax is solid and any solution obtained is viscous), and against high temperature extraction (when the wax is liquid but insoluble and the modifier is now insoluble).

The type of materials incorporated and their benefits include (excipients may display more than one of these properties): high melting point excipients resistant to heating and that prevent injecting; taste modifiers which prevent covert administration, snorting and dose dumping; water insolubles that are resistant to extraction and that prevent drink adulteration; waxy excipients that prevent snorting; viscosity modifiers resistant to dissolution and that prevent injecting and dose dumping; low density excipients that prevent drink adulteration; and dyes that disclose abuse of the pharmaceutical medicament. The contents of the abuse reististant capsule are typically solid at ambient temperature.

Examples of the above materials are: high melting point excipients - Poloxamer 188, PEG 8000; taste modifiers - denatonium benzoate, quinine sulphate; water insolubles - shellac, hexadecan-1-ol (cetyl alcohol); waxy excipients - glyceryl monostearate, hydrogenated cotton seed oil; viscosity modifiers - fumed silca (Aerosils™), hydroxy propyl methyl cellulose; low density excipients - Gelucires™; dyes - any food or pharma approved dyes (varies considerably between countries).

Formulations have been developed which address all of the common areas of abuse in both immediate and sustained release dosage forms.

In a first example there is provided an abuse resistant capsule, which has a gelatin outer shell capsule, and which contains temazepam pharmaceutical medicament and poloxamer 188 excipient modifier. In this example, the abuse resistant capsule is resistant to heating and therefore cannot be injected. In practice, the abuse resistant capsule melts at temperatures above 60°C, but re-solidifies when it comes into contact with a syringe at room temperature. This obviously makes it impossible to inject the contents of the abuse resistant capsule. Furthermore, if the abuse resistant capsule were to be injected, it would have to be injected at a temperature greater than 60°C, i.e., in the liquid state. A liquid of this temperature would be far too hot to inject into the human body.

Although in the example above the melting temperature is 65°C, the melting temperature can be any value from 35°C to 90°C. In fact, the upper temperature limit can be defined by the melting point of the hard shell outer capsule. The hard outer shell capsules withstand high filling temperatures and can be filled with various modifiers and pharmaceutical medicaments.

The hard shell outer capsule can further comprise modifiers and excipients and can also be made from hydroxypropyl methylcellulose (HPMC) or any other suitable material.

In a second example there is provided a method for preparing an abuse resistant capsule comprising the steps of:
preparing temazepam;
adding poloxamer 188; and
filling a gelatin hard shell outer capsule with the temazepam and the poloxamer 188;
wherein the poloxamer 188 is used to prevent the temazepam from being abused, and wherein the gelatin hard shell outer capsule is filled with a mixture of the temazepam and poloxamer 188 at a temperature greater than the melting point of the mixture, but less than 90°C.

Whilst in the example given above the temperature is 65°C it will be appreciated that any temperature at which the fill constituents are in liquid form would be suitable. Specifically, temperatures of between 35°C and 70°C are particularly useful.

In a second example there is provided an abuse resistant capsule comprising Flunitrazepam 0.2%, suspended in a mixture of Gelucire™ 44/14 (98.8%) and a pharma approved dye (1%).

This active is the compound used in 'date rape' and this formulation protects against covert administration because the main excipient Gelucire™ 44/14 is a non soluble wax (it is slowly dispersible and digestible), which is difficult to crush, with an unpleasant taste which has a density less than 1 and thus does not dissolve but floats on drinks. The incorporated dye will highlight the dosage unit and provide evidence of addition of a dosage unit to a drink.

In this case attempts to increase dissolution rates by powdering would fail as the formulation is a soft waxy material. This formulation would not dissolve quickly in a drink but would remain floating on the surface, highlighted by the dye incorporated. Any dissolution in the drink would both impart a colour and an unpleasant taste to the drink.

Overall this would impart considerable abuse resistance to this active material over that provided in current commercial dosage forms yet still perform normally when taken as intended.

In a third example there is provided an abuse resistant capsule comprising water soluble opiod drug 20%, paraffin wax 59%, hydroxyl propyl methyl cellulose (HPMC) 20%, taste modifier 1%.

In this case the dosage unit is most likely to be abused by a self abuser. The water soluble drug is protected by the insoluble paraffin wax however this can be released into the patient by 'channels' created by the dissolution of the drug substance and the HPMC. Attempts to 'snort' inject, extract or dose dump would be resisted in the following manner. 'Snorting' would be prevented as the formulation is soft and cannot be crushed to a powder. The taste modifier produces a very unpleasant taste in the mouth and nose.

Extraction or injection would be prevented at low temperature by the insolubility of paraffin wax in aqueous or common organic solvents (e.g. ethanol). Additionally any material dissolving would be rendered viscous by the HPMC. At high temperatures the HPMC is insoluble and would thus resist extraction. Similarly the paraffin wax would remain insoluble even if melted and would slow extraction.

Dose dumping would be resisted predominantly by the taste modifier however the inability to powder the formulation and its sticky, toffee like consistency requiring significant chewing to extract material would provide additional barriers to abuse.

The active ingredient may be present directly mixed with the modifier or modifiers or may be incorporated as separately formulated and prepared 'particles', e.g. granules, which incorporate the active in modifiers and are themselves suspended in additional modifiers. The mix of suspended 'particles' in modifiers is then filled into hard shell capsules to provide the abuse resistant dosage unit. The formulated 'particles' (100 -2000 micron in size) are comprised of active ingredient formulated with a modifier or modifier, as described for use in preparing abuse resistant dosage units, which are rendered into 'particles'. These 'particles' may be uncoated or may be coated with materials known to the art of 'particle' coating. This enables several levels of abuse resistance to be built into a dosage unit using the same range of modifiers.

In a fourth example is provided an abuse resistant capsule comprising a water soluble opioid drug (45%) suspended in hydrogenated castor oil (55%) which is rendered into 'particles' and coated with an enteric coating using materials and method as known in the art. The coated 'particles' (30%) are suspended in beeswax (50%), hydroxyl propyl methyl cellulose (18%) and fumed silica (2%) then filled as a liquid suspension into hard shell capsules.

In this case the dosage unit is most likely to be abused by a self abuser. The drug will release at the desired rate when used in the appropriate manner as the drug will diffuse out of the 'particles' then migrate through 'channels' created by the dissolution of the HPMC in the beeswax following the initial migration of alkaline media (from the intestine) inwards that breaks down the enteric barrier surrounding the 'particles'. Attempts to 'snort', inject, extract or dose dump would be resisted in the following manner. 'Snorting' would be prevented as the formulation is soft and cannot be crushed to a powder. Extraction would be prevented as the water soluble drug is protected from direct extraction in aqueous media or common organic solvents by the insoluble wax, hydrogenated castor oil, and is further protected from acid extraction by the enteric coat surrounding the 'particle. The drug is further protected from extraction after crushing or subdividing the dosage unit to enhance extraction by the beeswax which renders the dosage unit resistant to crushing and by the 'particles' which negate the effect of subdivision by e.g. cutting the dosage unit into smaller pieces does not increase extraction efficiency as the particles remain unchanged. Extraction at elevated temperature is resisted by the HPMC which becomes insoluble at elevated temperature. Injection is prevented by the inability to produce a concentrated extract or to directly melt and inject the formulated material due to its high melting point, high viscosity imparted by the beeswax and fumed silica and its 'particle' containing nature. Dose dumping by chewing is similarly resisted by the soft sticky consistency of the chewed mass and the inability to increase release by breaking up the 'particles'.

The abuse resistant capsule technology involves the application of hard shell liquid fill techniques using a combination of excipients to produce a dosage unit tailored to the required release profile, formulated from excipients chosen to provide the best deterrence to potential routes of abuse. The hard shell liquid fill may use a very wide range of excipients

The excipient can be chosen from one or more of the materials selected from the following groups; consisting of:
thermosoftening pharmaceutical bases including waxes, poloxamers, macrogol glycerides, PEGs, glycerol monooleates or monostearates, hydrogenated or partially hydrogenated glycerides and hard fats such as beeswax, poloxamer 188, Gelucires™, polyethylene 6000, glycerol monostearate, hydrogenated palm kernel oil, hydrogenated cottonseed oil, Softisan™ 138, Gelucire 40/01™ hexadecan-1-ol; Thixotropes such as fumed silica and pulverised attapulgite and viscosity modifiers such as hydroxyl propyl methyl cellulose or Gellan gum™ to increase viscosity or the standard pharmaceutical or food grade oils such as fractionated coconut oil, soyabean oil etc to decrease viscosity.

Also, it will be appreciated that the excipient can be any material or compound that confers the desired properties to the abuse resistant capsule and that more than one modifier can be used in any single embodiment of the invention.

The abuse resistant capsules can contain modifiers that are high point melting solids, which are digestible but water insoluble, thereby reducing the potential to 'dose dump' by chewing multiple dosage units. The modifiers can be waxy solids which do not melt at 'injectable' temperatures or dissolve readily in water, and that cannot be crushed to a powder. These properties are conferred to the anti-abuse pharmaceutical medicament. The modifiers can even have the above features and, additionally, be of low enough density that they will float on top of water based drinks. Abuse resistant capsules incorporating modifiers of this type prevent the adulteration of drinks, resulting in the abuse resistant capsules floating, yet not dissolving, on the surface of the drink. Furthermore, the abuse resistant capsule can contain up to 50% solid materials.

The capsules can also be adapted to provide additional abuse resistance by the addition of excipients that naturally have a strong taste, or by the incorporation of modifiers chosen specifically for their unpleasant flavour. When abuse resistant capsules of this type are added to drinks (a process known as "spiking"), the "spiked" drinks are immediately rejected, owing to their unpleasant taste. Even in dim lighting and under the effects of alcohol (both of which can mask simple coloured warning agents in tablets) potential victims reject drinks spiked with abuse resistant capsules of this type.

Thixotropes can be also be incorporated in the abuse resistant capsules to provide additional resistance to abuse by injection. Attempts to produce an injectable solution using the abuse resistant capsules containing thixotropes results in the formation of a highly viscous mass, which is not injectable.

Excipients can be chosen to resist extraction by either resisting dissolution in the abuser's solvent of choice (e.g water, alcohol, household chemicals) or resisting abuse by matching the solubility properties of the active pharmaceutical medicament and thus make separation of the pharmaceutical medicament and the excipient mass difficult.

Although in the examples only one pharmaceutical medicament is included in the abuse resistant capsule, it will be appreciated that a mixture of medicaments may, where appropriate, be used.

The above gives some illustration of how abuse resistance can be built into hard shell liquid fill dosage units. However, it will be appreciated that the exact resistance features incorporated are selected to combat the likely routes of abuse of the particular pharmaceutical medicament.

## Claims

1. An abuse resistant capsule comprising a hard shell liquid filled with a pharmaceutical medicament and at least one modifier selected to prevent abuse of the pharmaceutical medicament, wherein at least one modifier is a waxy substance having a melting point of greater than 50°C, wherein said modifier is present in an amount greater than 10% of the hard shell liquid filling.

2. An abuse resistant capsule as claimed in Claim 1 where the at least one modifier is insoluble in liquid.

3. An abuse resistant capsule as claimed in Claim 2, where the at least one modifier is insoluble in aqueous solvents.

4. An abuse resistant capsule as claimed in Claims 2 to 3, where the at least one modifier is insoluble in ethanol.

5. An abuse resistant capsule as claimed in Claims 2 to 4, where the at least one modifier has a density less than 1.

6. An abuse resistant capsule as claimed in the preceding Claims where the hard shell outer capsule is gelatin or hydroxypropyl methylcellulose (HPMC).

7. An abuse resistant capsule as claimed in the preceding Claims where the at least one modifier is a thermosoftening pharmaceutical base selected from the group consisting of: waxes, poloxamers, macrogol glycerides, PEGs, glycerol monooleates or monostearates, hydrogenated or partially hydrogenated glycerides and hard fats such as beeswax, poloxamer 188, polyethylene 6000, glycerol monostearate, hydrogenated palm kernel oil, hydrogenated cottonseed oil.

8. An abuse resistant capsule as claimed in the preceding Claims additionally comprising a thixotrope selected from the group consisting of fumed silica and pulverised attapulgite and hydroxypropyl methylcellulose (HPMC).

9. An abuse resistant capsule as claimed in the preceding Claims additionally comprising a viscosity modifier selected from the group consisting of hydroxyl propyl methyl cellulose or Gellan gum, fractionated coconut oil, soyabean oil.

10. An abuse resistant capsule as claimed in the preceding Claims where the at least one modifier comprises a digestible material.

11. An abuse resistant capsule as claimed in the preceding Claims where the at least one modifier comprises suspended solids.

12. An abuse resistant capsule as claimed in Claim 11, where the modifier and pharmaceutical medicament are incorporated in the abuse resistant capsule as particles suspended in one or more additional modifiers, preferably where the particles are 100-2000 micron in size.

13. A method for preparing an abuse resistant capsule as Claimed in Claims 1 to 12 comprising the steps of:
a) preparing a pharmaceutical medicament;
b) adding at least one modifier; and
c) liquid filling a hard shell with the pharmaceutical medicament and the at least one modifier
where the modifier is selected to prevent abuse of the pharmaceutical medicament, wherein the hard shell is filled at a temperature of above 50°C, and wherein said modifier is present in an amount greater than 10% of the liquid filling.

14. A method for preparing an abuse resistant capsule as claimed in Claim 13, where the method comprises the further step of heating the pharmaceutical medicament to above 50°C prior to filling.

15. A method for preparing an abuse resistant capsule as claimed in Claims 13 to 14, where the pharmaceutical medicament and at least one modifier are liquid at the filling temperature.

## Patentansprüche

1. Missbrauchssichere Kapsel, umfassend eine harte Kapselhülle, die mit einem pharmazeutischen Medikament und mindestens einem Modifikator flüssiggefüllt ist, der ausgewählt ist, damit der Missbrauch des pharmazeutischen Medikaments verhindert wird, wobei mindestens ein Modifikator eine wachsartige Substanz mit einem Schmelzpunkt von mehr als ungefähr 50°C ist, wobei der Modifikator in einer Menge von mehr als 10% der Flüssigkeitsfüllung der harten Kapselhülle zugegen ist.

2. Missbrauchssichere Kapsel nach Anspruch 1, wobei der mindestens eine Modifikator in Flüssigkeit unlöslich ist.

3. Missbrauchssichere Kapsel nach Anspruch 2, wobei der mindestens eine Modifikator in wässrigen Lösungsmitteln unlöslich ist.

4. Missbrauchssichere Kapsel nach den Ansprüchen 2 bis 3, wobei der mindestens eine Modifikator in Ethanol unlöslich ist.

5. Missbrauchssichere Kapsel nach den Ansprüchen 2 bis 4, wobei der mindestens eine Modifikator eine Dichte von weniger als 1 aufweist.

6. Missbrauchssichere Kapsel nach den vorhergehenden Ansprüchen, wobei die äußere Kapsel der Hartkapsel Gelatine oder Hydroxypropylmethylcellulose (HPMC) ist.

7. Missbrauchssichere Kapsel nach den vorhergehenden Ansprüchen, wobei der mindestens eine Modifikator eine durch Wärme erweichende pharmazeutische Basis ist, ausgewählt aus der Gruppe bestehend aus Wachsen, Poloxameren, Makrogolglyceriden, PEGs, Glycerinmonooleaten oder -monostearaten, hydrierten oder teilweise hydrierten Glyceriden und harten Fetten wie Bienenwachs, Poloxamer 188, Polyethylen 6000, Glycerinmonostearat, hydriertem Palmkernöl, hydriertem Baumwollsamenöl.

8. Missbrauchssichere Kapsel nach den vorhergehenden Ansprüchen, zudem umfassend ein Thixotrop, das aus der Gruppe ausgewählt ist, die aus pyrogener Kieselsäure und pulverisiertem Attapulgit und Hydroxypropylmethylcellulose (HPMC) besteht.

9. Missbrauchssichere Kapsel nach den vorhergehenden Ansprüchen, umfassend einen Viskositätsmodifikator, der aus der Gruppe ausgewählt ist, die aus Hydroxylpropylmethylcellulose oder Gellangummi, fraktioniertem Kokosnussöl und Sojaöl besteht.

10. Missbrauchssichere Kapsel nach den vorhergehenden Ansprüchen, wobei der mindestens eine Modifikator ein verdauliches Material umfasst.

11. Missbrauchssichere Kapsel nach den vorhergehenden Ansprüchen, wobei der mindestens eine Modifikator suspendierte Feststoffe umfasst.

12. Missbrauchssichere Kapsel nach Anspruch 11, wobei der Modifikator und das pharmazeutische Medikament in die missbrauchssichere Kapsel als Partikel eingebracht sind, die in einem oder mehreren zusätzlichen Modifikatoren suspendiert sind, wobei die Partikel vorzugsweise eine Größe von 100 bis 2000 Mikron haben.

13. Verfahren zur Herstellung einer missbrauchssicheren Kapsel nach den Ansprüchen 1 bis 12, umfassend die Schritte:
a) Herstellen eines pharmazeutischen Medikaments;
b) Hinzufügen mindestens eines Modifikators; und
c) Flüssigbefüllen einer harten Kapselhülle mit dem pharmazeutischen Medikament und dem mindestens einen Modifikator,
wobei der Modifikator ausgewählt ist, damit der Missbrauch des pharmazeutischen Medikaments verhindert wird, wobei die harte Kapselhülle bei einer Temperatur von über 50°C befüllt wird und wobei der Modifikator in einer Menge von mehr als 10% der Flüssigkeitsfüllung der harten Kapselhülle zugegen ist.

14. Verfahren zur Herstellung einer missbrauchssicheren Kapsel nach Anspruch 13, wobei das Verfahren den weiteren Schritt umfasst, bei dem das pharmazeutische Medikament vor dem Befüllen über 50°C erwärmt wird.

15. Verfahren zur Herstellung einer missbrauchssicheren Kapsel nach den Ansprüchen 13 bis 14, wobei das pharmazeutische Medikament und mindestens ein Modifikator bei der Abfülltemperatur flüssig sind.

## Revendications

1. Capsule anti-abus comprenant un liquide de coque dure remplie d'un médicament pharmaceutique et d'au moins un modificateur sélectionné pour prévenir les abus du médicament pharmaceutique, dans laquelle au moins un modificateur est une substance cireuse ayant un point de fusion supérieur à 50 °C, dans laquelle ledit modificateur est présent en une quantité supérieure à 10 % du remplissage liquide de coque dure.

2. Capsule anti-abus selon la revendication 1, où l'au moins un modificateur est insoluble dans le liquide.

3. Capsule anti-abus selon la revendication 2, où l'au moins un modificateur est insoluble dans des solvants aqueux.

4. Capsule anti-abus selon les revendications 2 à 3, où l'au moins un modificateur est insoluble dans l'éthanol.

5. Capsule anti-abus selon les revendications 2 à 4, où l'au moins un modificateur a une densité inférieure à 1.

6. Capsule anti-abus selon les revendications précédentes, où la capsule externe à coque dure est la gélatine ou l'hydroxypropylméthylcellulose (HPMC).

7. Capsule anti-abus selon les revendications précédentes, où l'au moins un modificateur est une base pharmaceutique thermoplastique choisie dans le groupe constitué de : cires, poloxamères, glycérides de macrogol, PEG, monooléates ou monostéarates de glycérol, glycérides hydrogénés ou partiellement hydrogénés et graisses dures telles que la cire d'abeille, le poloxamère 188, le polyéthylène 6000, le monostéarate de glycérol, l'huile de palmiste hydrogénée, l'huile de coton hydrogénée.

8. Capsule anti-abus selon les revendications précédentes, comprenant en outre un thixotrope choisi dans le groupe constitué de la silice pyrogénée et l'attapulgite pulvérisée et l'hydroxypropylméthylcellulose (HPMC).

9. Capsule anti-abus selon les revendications précédentes, comprenant en outre un modificateur de viscosité choisi dans le groupe constitué de l'hydroxypropylméthylcellulose ou la gomme gellane, l'huile de noix de coco fractionnée, l'huile de soja.

10. Capsule anti-abus selon les revendications précédentes, où l'au moins un modificateur comprend un matériau digestible.

11. Capsule anti-abus selon les revendications précédentes, où l'au moins un modificateur comprend des solides en suspension.

12. Capsule anti-abus selon la revendication 11, où le modificateur et le médicament pharmaceutique sont incorporés dans la capsule anti-abus sous forme de particules en suspension dans un ou plusieurs modificateurs supplémentaires, de préférence où les particules ont une taille de 100 à 2000 microns.

13. Procédé de préparation d'une capsule anti-abus selon les revendications 1 à 12 comprenant les étapes de :
a) préparation d'un médicament pharmaceutique ;
b) ajout d'au moins un modificateur ; et
c) remplissage de liquide d'une coque dure avec le médicament pharmaceutique et l'au moins un modificateur
où le modificateur est sélectionné de façon à prévenir l'abus du médicament pharmaceutique, dans lequel la coque dure est remplie à une température supérieure à 50 °C, et dans lequel ledit modificateur est présent en une quantité supérieure à 10 % du remplissage liquide.

14. Procédé de préparation d'une capsule anti-abus selon la revendication 13, où le procédé comprend l'étape supplémentaire de chauffage du médicament pharmaceutique au-dessus de 50 °C avant remplissage.

15. Procédé de préparation d'une capsule anti-abus selon les revendications 13 à 14, où le médicament pharmaceutique et au moins un modificateur sont liquides à la température de remplissage.
